# EUROPÄISCHE PATENTSCHRIFT

(11) **EP 1 005 447 B1**
(45) Veröffentlichungstag und Bekanntmachung des Hinweises auf die Patenterteilung: **23.04.2003**
(21) Anmeldenummer: 98945254.5
(22) Anmeldetag: 17.08.1998
(51) Int. Cl.: C07C 253/00, C07C 255/25, C07C 229/16, C07C 229/08, C07D 233/74

(54) **VERFAHREN ZUR HERSTELLUNG VON LANGKETTIGEN GLYCIN-N,N-DIESSIGSÄURE-DERIVATEN**
METHOD FOR PRODUCING LONG-CHAIN GLYCINE-N,N-DIACETIC ACID DERIVATIVES
PROCEDE DE PRODUCTION DE DERIVES D'ACIDE DIACETIQUE-GLYCIN-N,N- A LONGUE CHAINE

(30) Priorität: 21.08.1997 DE 19736476
(43) Veröffentlichungstag der Anmeldung: 07.06.2000
(73) Patentinhaber: BASF AKTIENGESELLSCHAFT, 67056 Ludwigshafen (DE)
(72) Erfinder: RAHM, Rainer, D-67071 Ludwigshafen (DE); GREINDL, Thomas, D-67098 Bad Dürkheim (DE); OFTRING, Alfred, D-67098 Bad Dürkheim (DE); OETTER, Günter, D-67227 Frankenthal (DE); DETERING, Jürgen, D-67117 Limburgerhof (DE); BRAUN, Gerold, D-67071 Ludwigshafen (DE)
(74) Vertreter: Isenbruck, Günter, Dr.
(86) Internationale Anmeldenummer: EP9805220
(87) Internationale Veröffentlichungsnummer: WO99010314

(56) Entgegenhaltungen:
- EP-A- 0 745 582
- WO-A-94/29421
- HIROYUKI MINAMIKAWA ET AL.: "Asymmetric Hydrocyanation of Aldehydes Using Chiral Titanium Reagents" BULLETIN OF THE CHEMICAL SOCIETY OF JAPAN., Bd. 61, Nr. 12, Dezember 1988, Seiten 4379-4383, XP002100390 TOKYO JP
- CHEMICAL ABSTRACTS, vol. 70, no. 11, 17. März 1969 Columbus, Ohio, US; abstract no. 46856g, MICHIO NAKANISHI ET AL.: "Higher unsaturated aliphatic amino acids" Seite 270; Spalte 2; XP002100391 & JP 68 012567 A (YOSHITOMI PHARMACEUTICAL) 28. Mai 1968

## Beschreibung

Die Erfindung betrifft Verfahren zur Herstellung von langkettigen Glycin-N,N-diessigsäure-Derivaten.

Glycin-N,N-diessigsäure-Derivate können als biologisch abbaubare Komplexbildner für Erdalkali- und Schwermetallionen eingesetzt werden.

Unterschiedliche Verfahren zur Herstellung dieser Verbindungen sind bekannt.

In der WO 94/29421 sind Verfahren zur Herstellung von Glycin-N,N-diessigsäure-Derivaten beschrieben. Dabei werden langkettige aliphatische Aldehyde mit Iminodiacetonitril und HCN zu Alkylglycinnitril-N,N-diacetonitril umgesetzt, wobei die erhaltene Verbindung hydrolysiert wird. Die Verbindungen können ebenfalls erhalten werden durch Umsetzung der Aldehyde mit HCN und Ammoniak zu substituierten Aminonitrilen, die zu substituierten Aminosäuren hydrolysiert werden, worauf eine Umsetzung mit Formaldehyd und Natriumcyanid folgt. Für manche Verbindungen ist die Verfahrensführung zu aufwendig, da lange Reaktionszeiten erforderlich sind. Auch die Ausbeuten für die gewünschten Verbindungen sind noch verbesserungsfähig. Die vorgeschlagenen Verfahren eignen sich nicht immer für die Übertragung in den großtechnischen Maßstab.

In der DE-A-195 18 986 ist ein Verfahren zur Herstellung von Glycin-N,N-diessigsäure-Derivaten durch Umsetzung von Glycinderivaten oder deren Vorstufen mit Formaldehyd und Cyanwasserstoff oder von Iminodiacetonitril oder Iminodiessigsäure mit entsprechenden Aldehyden und Cyanwasserstoff in wäßrig-saurem Medium beschrieben. Als Ausgangsmaterial werden dabei aus der technischen Synthese von Glycinderivaten oder deren Vorstufen oder von Iminodiacetonitril oder Iminodiessigsäure stammendes nicht gereinigtes Rohmaterial oder bei solchen Synthesen anfallende Mutterlaugen eingesetzt. Die Verfahrensführung erfolgt wie in WO 94/29421 angegeben.

Die DE-A-195 18 187 betrifft Verfahren zur Herstellung von Glycin-N,N-diessigsäure-Derivaten durch Umsetzung von Glycinderivaten oder deren Vorstufen mit Formaldehyd und Alkalimetallcyanid in wäßrig-alkalischem Medium. Das Verfahren basiert ebenfalls auf dem in WO 94/29421 beschriebenen Verfahren, jedoch gibt man zunächst 0,5 bis 30% der zur Umsetzung benötigten Menge an Alkalimetallcyanid zu den vorgelegten Glycinderivaten oder deren Vorstufen und dosiert anschließend die restliche Menge Alkalimetallcyanid und den Formaldehyd gleichzeitig über einen Zeitraum von 0,5 bis 12 Stunden zu. Auch diese Verfahrensvariante kann nicht alle der vorstehend aufgeführten Nachteile beseitigen.

Aufgabe der vorliegenden Erfindung ist die Bereitstellung eines Verfahrens zur Herstellung von Glycin-N,N-diessigsäure-Derivaten durch Umsetzung von Iminodiacetonitril mit Aldehyden und HCN oder Alkalicyaniden, das die vorstehend aufgeführten Nachteile vermeidet und fiir eine Übertragung in den großtechnischen Maßstab geeignet ist. Das Verfahren soll in kurzen Reaktionszeiten zu hohen Ausbeuten führen; zudem sollen alternative Verfahren bereitgestellt werden, die die vorstehend aufgeführten Nachteile vermeiden.

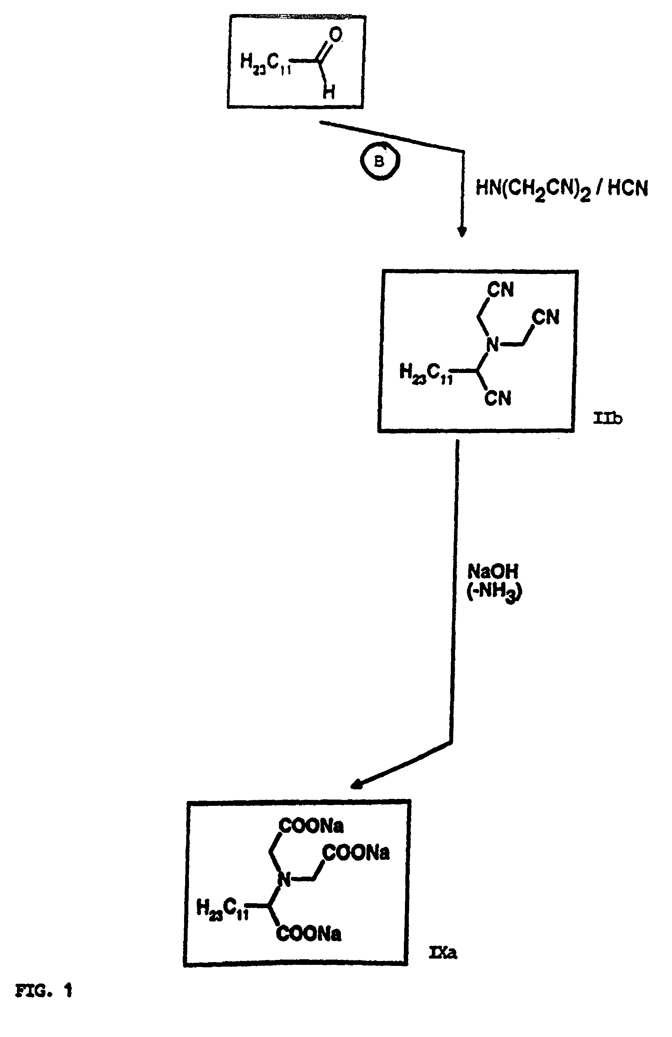

Die erfindungsgemäßen Verfahren werden anhand der in der Zeichnung dargestellten Reaktionsschemata erläutert, wobei
Figur 1 Reaktionsschema B anhand von Dodecanal als Beispiel zeigt und Insbesondere wird die Aufgabe gelöst durch ein Verfahren zur Herstellung von Verbindungen der allgemeinen Formel IIb 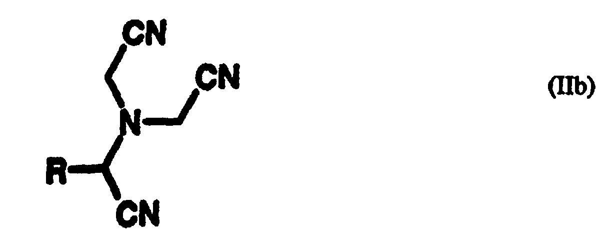 in der R für C₆ bis C₃₀-Alkyl oder C₆ bis C₃₀-Alkenyl, welche zusätzlich als Substituenten bis zu 5 Hydroxylgruppen, Formylgruppen, C₁ bis C₄-Alkoxygruppen, Phenoxygruppen oder C₁ bis C₄-Alkoxycarbonylgruppen tragen können und durch bis zu 5 nicht benachbarte Sauerstoffatome unterbrochen sein können, oder Alkoxylat-Gruppierungen der Formel -(CH₂)ₖ-O-(A¹O)ₘ-(A²O)ₙ - Y steht, in der A¹ und A² unabhängig voneinander 1,2-Alkylengruppen mit 2 bis 4 C-Atomen bezeichnen, Y Wasserstoff, C₁ bis C₁₂-Alkyl, Phenyl oder C₁- bis C₄-Alkoxycarbonyl bedeuten und k für die Zahl 1, 2 oder 3 sowie m und n jeweils für Zahlen von 0 bis 50 stehen, wobei die Summe aus m + n mindestens 4 betragen muß,
durch Umsetzung von Iminodiacetonitril mit Aldehyden der allgemeinen Formel R-CHO und HCN oder Alkalicyaniden, wobei das Verfahren
a) ohne Mitverwendung eines organischen Lösungsmittels durch umsetung in substanz und in Anwesenheit einer Lewis- oder Brönsted-Säure durchgeführt wird.

Die Umsetzung von Iminodiacetonitril mit Aldehyden und HCN kann beschleunigt werden durch Umsetzung ohne Mitverwendung eines organischen Lösungsmittels und insbesondere auch ohne Mitwerwendung von weiterem Wasser im Reaktionssystem, das heißt durch Umsetzung in Substanz und in Anwesenheit einer Lewis- oder Brönsted-Säure.

Die Umsetzung kann außerdem in Anwesenheit eines Emulgators, der vorzugsweise in einer Konzentration von 1 bis 50 g/l, besonders bevorzugt 2 bis 30 g/l an Reaktionsgemisch eingesetzt wird, durchgeführt werden. Als Emulgatoren können alle dafür geeigneten Verbindungen eingesetzt werden. Beispiele sind anionische, kationische, amphotere und nichtionische Emulgatoren. Als lipophile Endgruppen weisen die Emulgatoren dabei in der Regel geradkettige oder verzweigte Alkylreste, die ungesättigte Bindungen enthalten können, Arylreste oder Alkylarylreste auf. Als hydrophile Endgruppen kommen bei anionischen Emulgatoren beispielsweise Carboxylat, Sulfonat, Sulfat, Phosphat, Polyphosphat, Lactat, Citrat und Tartrat in Betracht. Für kationische Emulgatoren kommen beispielsweise Aminsalze und quarternäre Ammoniumverbindungen in Betracht. Für amphotere Emulgatoren kommen zwitterionische Verbindungen nach Art von Aminosäuren und beispielsweise Betain in Betracht. Für nichtionische Emulgatoren kommen Reste von Alkoholen, Polyethern, Glycerin, Sorbit, Pentaerythrit, Saccharose, Essigsäure oder Milchsäure in Betracht. Die Emulgatoren können zudem hydrophile Zwischengruppen, wie Hydroxyl-, Ester-, Sulfamid-, Amid-, Polyamid-, Polyamin-, Amin-, Ether-, Polyether-, Glycerin-, Sorbit-, Pentaerythrit- oder Saccharosegruppen aufweisen.

Beispiele geeigneter Emulgatoren sind Ethoxylierungsprodukte und Fettsäurekondensationsprodukte, Fettalkoholethoxylate und gegebenenfalls Polyglykole und Umsetzungsprodukte von Phenolen und Ölen mit Ethylenoxid.

Insbesondere werden als Emulgatoren Verbindungen wie Alkalialkylsulfate, insbesondere Natriumdodecylsulfat oder Gemische von hydrophoben Alkylsulfaten eingesetzt. Auch der Einsatz von nichtionischen Tensiden, wie Fettalkoholethoxylaten, die insbesondere schaumarm sind, ist möglich.

Der vorstehend angegebene Verfahrensschritt bezieht sich auf das Verfahren B fiir die Umsetzung des Ausgangsaldehyds zur Verbindung der allgemeinen Formel IIb.

Die Erfindung betrifft weiterhin ein Verfahren zur Herstellung von Verbindungen der allgemeinen Formel IX 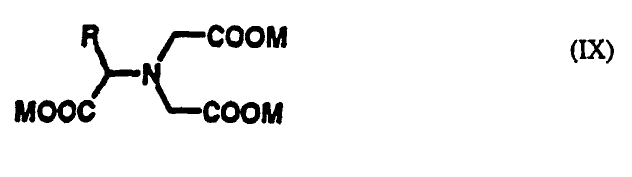 in der R die vorstehend angegebene Bedeutung hat und M Wasserstoff, Alkalimetall, Erdalkalimetall, Ammonium oder substituiertes Ammonium in den entsprechend stöchiometrischen Mengen bedeutet, durch Umsetzung von Verbindungen der allgemeinen Formel IIb, wie sie vorstehend definiert sind und die nach dem vorstehenden Verfahren hergestellt wurden, mit Alkalihydroxidlösungen bei einem Druck in einem Bereich, von 1 bis 40 bar und gegebenenfalls mit Lösungen, die Ionen von M enthalten. Dieser Umsetzung entspricht der Verfahrensschritt von der Verbindung von der allgemeinen Formel IIb zur Verbindung der allgemeinen Formel IXa in Figur 1.

Die vorstehend aufgeführten Verfahren können zudem durch Anwendung einer oder mehrerer der folgenden Verfahrensvarianten verbessert werden: Mitverwendung von Schaumdämpfern, Mitverwendung von Emulgatoren, Temperatur im Bereich von 20 bis 220°C, vorzugsweise 30 bis 180°C, insbesondere 50 bis 140°C, pH-Wert im Bereich von 0 bis 11. Werden die Umsetzungen unter Druck durchgeführt, so beträgt der Druck vorzugsweise 1,5 bis 30 bar, insbesondere 2 bis 15 bar.

Als Alkalimetall-, Ammonium- und substituierte Ammoniumsalze eignen sich vor allem die Natrium-, Kalium- und Ammoniumsalze, insbesondere bei den Verbindungen der allgemeinen Formel IX das Trinatrium-, Trikalium- und Triammoniumsalz, sowie organische Triaminsalze mit einem tertiären Stickstoffatom.

Als den organischen Aminsalzen zugrundeliegende Basen kommen insbesondere tertiäre Amine, wie Trialkylamine mit 1 bis 4 C-Atomen im Alkyl, wie Trimethyl- und Triethylamin, und Trialkanolamine mit 2 oder 3 C-Atomen im Alkanolrest, bevorzugt Triethanolamin, Tri-n-propanolamin oder Triisopropanolamin, in Betracht.

Als Erdalkalimetallsalze werden insbesondere die Calcium- und Magnesiumsalze eingesetzt.

Für den Rest R kommen als geradkettige oder verzweigte Alk(en)ylreste vor allem C₆- bis C₂₀-Alkyl und -Alkenyl, hierbei insbesondere geradkettige, von gesättigten oder ungesättigten Fettsäuren abgeleitete Reste, in Betracht. Beispiele für einzelne Reste R sind: n-Hexyl, n-Heptyl,. 3-Heptyl (abgeleitet von 2-Ethylhexansäure), n-Octyl, iso-Octyl (abgeleitet von iso-Nonansäure), n-Nonyl, n-Decyl, n-Undecyl, n-Dodecyl, iso-Dodecyl (abgeleitet von iso-Tridecansäure), n-Tridecyl, n-Tetradecyl, n-Pentadecyl, n-Hexadecyl, n-Heptadecyl, n-Octadecyl, n-Nonadecyl, n-Eicosyl und n-Heptadecenyl (abgeleitet von Ölsäure). Es können für R auch Gemische auftreten, insbesondere solche, die sich von natürlich vorkommenden Fettsäuren und von synthetisch erzeugten technischen Säuren, beispielsweise durch Oxosynthese, ableiten.

Als C₁- bis C₁₂-Alkylen-Brücken A dienen vor allem Polymethylengruppierungen der Formel ―(CH₂)ₖ―, worin k eine Zahl von 2 bis 12, insbesondere von 2 bis 8 bezeichnet, das heißt 1,2-Ethylen, 1,3-Propylen, 1,4-Butylen, Pentamethylen, Hexamethylen, Heptamethylen, Octamethylen, Nonamethylen, Decamethylen, Undecamethylen und Dodecamethylen. Hexamethylen, Octamethylen, 1,2-Ethylen und 1,4-Butylen werden hierbei besonders bevorzugt. Daneben können aber auch verzweigte C₁- bis C₁₂-Alkylengruppen auftreten, zum Beispiel ―CH₂CH(CH₃)CH₂―, ―CH₂C(CH₃)₂CH₂―, ―CH₂CH(C₂H₅) ― oder ―CH₂CH(CH₃) ―.

Die C₆- bis C₃₀-Alkyl- und C₆- bis C₃₀-Alkenylgruppen können bis zu 5, insbesondere bis zu 3 zusätzliche Substituenten der genannten Art tragen und durch bis zu 5, insbesondere bis zu 3 nicht benachbarte Sauerstoffatome unterbrochen sein. Beispiele für solche substituierten Alk(en)ylgruppen sind -CH₂OH, -CH₂CH₂OH, -CH₂CH₂-O-CH₃, -CH₂CH₂-O-CH₂CH₂-O-CH₃, -CH₂-O-CH₂CH₃, -CH₂-O-CH₂CH₂-OH, -CH₂-CHO, -CH₂-OPh, -CH₂-COOCH₃ oder -CH₂CH₂-COOCH₃.

Als Alkoxylat-Gruppierungen kommen insbesondere solche in Betracht, bei denen m und n jeweils für Zahlen von 0 bis 30, vor allem von 0 bis 15 stehen. A¹ und A² bedeuten von Butylenoxid und vor allem von Propylenoxid und von Ethylenoxid abgeleitete Gruppen. Von besonderem Interesse sind reine Ethoxylate und reine Propoxylate, aber auch Ethylenoxid-Propylenoxid-Blockstrukturen können auftreten.

Die eingesetzten langkettigen Monoaldehyde in Variante B entstammen vorzugsweise großtechnisch durchführbaren Verfahren zu deren Herstellung aus leicht verfügbaren und billigen Grundchemikalien, insbesondere Hydroformylierungsreaktionen der entsprechenden α-Olefine und metallkatalysierten Reduktionsreaktionen der zugrundeliegenden Carbonsäuren und -ester.

Bei einzelnen Varianten der vorstehend und nachstehend beschriebenen Verfahren kann das Auftreten von Schäumen insbesondere auf der Verseifungsstufe durch das entstehende Ammoniak Probleme bereiten. Der Zusatz von geringen bis sehr geringen Mengen eines Schaumdämpfers, vorzugsweise eines Siliconentschäumers, führt zum Zusammenbruch des Schaumes oder zur Reduktion auf ein Minimum, das verfahrenstechnisch gut zu beherrschen ist. Auch andere schaumdämpfende Substanzen, wie Fettalkoholkethoxilate, Phosphorsäureester, etc. können eingesetzt werden. Der Zusatz von Tensiden, wie Alkoholalkoxilaten, ist besonders vorteilhaft, da diese nach erfolgter Umsetzung im Produkt verbleiben und im Gemisch eingesetzt werden können.

Die freien Säuren der Verbindungen IX können, falls die Verbindungen IX als Salze anfallen, durch Ansäuern nach üblichen Methoden erhalten werden.

### Variante B

Die erfindungsgemäße Umsetzung von Iminodiacetonitril, Iminodiessigsäure oder deren Derivaten, insbesondere Iminodiessigsäureestern oder -amiden mit den entsprechenden langkettigen Aldehyden und Cyanwasserstoff gemäß Ausführungsform B erfolgt durch Umsetzung von rohem Iminodiacetonitril mit dem Aldehyd und Blausäure zum entsprechenden Glycinnitril-N,N-diacetonitril und anschließende alkalische Hydrolyse zu den Verbindungen IX bei einem Druck von 1 bis 40 bar, insbesondere 2 bis 15 bar und Temperaturen zwischen 20 und 220°C, insbesondere 50 und 140°C Auch höhere Drücke, zum Beispiel durch Aufpressen von Schutzgas, wie Stickstoff, sind möglich.

Die Verseifung von nach erfolgter Umsetzung vorliegender Nitrilfunktionen zu Carboxylatgruppen wird üblicherweise mit 0,8 bis 2,0, insbesondere 1,0 bis 1,5 Moläquivalenten pro Nitrilfunktion wäßriger Natron- oder Kalilauge oder alkoholischer Natron- oder Kalilauge, wie ethanolischer Natron- oder Kalilauge mit einem Alkoholgehalt zwischen 5 und 50%, bei einem Druck von insbesondere 2 und 15 bar und Temperaturen zwischen 20 und 220°C, insbesondere 50 und 140°C durchgeführt. Auch höhere Drücke, zum Beispiel durch Aufpressen von Schutzgas, wie beispielsweise Stickstoff, sind möglich. Es hat sich als besonders günstig herausgestellt, den entstehenden Ammoniak kontinuierlich über ein Überströmventil bei Erreichen eines vorgegebenen Druckes von 1 bis 10 bar, insbesondere 2 bis 6 bar, auf den vorgegebenen Druck zu entspannen.

Als Nebenprodukte finden sich in den jeweiligen Verseifungslösungen gelegentlich geringe Mengen der entsprechenden α-Hydroxicarbonsäuren und Fettsäuren, welche bei der Hydrolyse von nichtumgesetzten Cyanhydrin oder intermediär aus Aldehyd und Cyanwasserstoff gebildetem Cyanhydrin entstehen. Da diese Verbindungen die Produkteigenschaften positiv beeinflussen, brauchen sie nicht aus den Reaktionsgemischen entfernt zu werden, weshalb teure und aufwendige Reinigungsschritte entfallen. müssen vorteilhafterweise nicht abgetrennt werden, da sie die Produkteigenschaften positiv beeinflussen oder ergänzen können.

Besonders bevorzugt ist das Herstellungsverfahren in Substanz,das heißt in der Schmelze ohne Verwendung von zusätzlichen Lösungsmitteln. Dabei werden vorzugsweise die vorstehend genannten Emulgatoren eingesetzt. Die Verseifung erfolgt vorzugsweise unter erhöhtem Druck, wobei bei Überschreiten eines höchstzulässigen Drucks das Reaktionsgemisch auf den gewünschten Druck entspannt wird. Die bevorzugten Verfahren werden nachstehend anhand der Beispiele detaillierter erläutert. Dort sind auch die bevorzugten Alkohole angegeben.

Die Reaktionsführung kann auch entsprechend der in DE-A-195 18 987 beschriebenen Dosieranleitungen erfolgen. Weitere Verfahrensweisen können der WO 94/29421 entnommen werden.

Die Erfindung wird nachstehend anhand von Beispielen näher erläutert.

### Beispiel 1 (Referenz)

### D,L-n-Undecylglycinnitril-N.N-diacetonitril

Eine Suspension von 71,3 g (0,75 mol) 99 %igem Iminodiacetonitril in 150 g Wasser und 100 g Methanol wird in einem Stahlautoklaven mit 7,5 g 50 %iger Schwefelsäure auf pH = 1,0 eingestellt. Dazu werden bei Raumtemperatur 31 g (1,14 mol) Blausäure getropft, bevor der Autoklav verschlossen und auf 35 bis 40°C erwärmt wird. 142,5 g (0,75 mol) 97%iger Laurinaldehyd in 50 g Methanol werden mit Hilfe einer Membranpumpe innerhalb von 1,5 h zudosiert. Es wird auf 100°C hochgeheizt und 8 Stunden bei der gleichen Temperatur und einem Druck von ca. 3,5 bar gerührt, bis durch Titration keine Änderung des Blausäuregehaltes mehr feststellbar ist. Nach Abkühlen auf 10°C wird die Phase, die das gebildete D,L-n-Undecylglycinnitril-N,N-diacetonitril enthält, von der wäßrigen Phase abgetrennt und von allen flüchtigen Bestandteilen befreit. Nach Umkristallisieren aus wenig Ethanol werden 192,7 g (0,67 mol; 89% d. Th.) Trinitril erhalten, dessen Reinheit mittels Gaschromatographie bestimmt wird.

### Beispiel 2

### D,L-n-Undecylglycinnitril-N,N-diacetonitril

48 g (0,5 mol) 99%iges Iminodiacetonitril, 95 g (0,5 mol) 97%iger Laurinaldehyd, 1,6 g p-Toluolsulfonsäuremonohydrat und 19 g (0,7 mol) Blausäure werden bei Raumtemperatur im Kolben mit Intensivkühler vorgelegt. Es wird langsam auf 70°C hochgeheizt und 3 Stunden bei dieser Temperatur nachgerührt, bis durch Titration keine Änderung des Blausäuregehaltes mehr feststellbar ist. Das rohe Reaktionsgemisch wird in Wasser gegeben, die organische Phase abgetrennt und aus wenig Ethanol umkristallisiert. Die Ausbeute an D,L-Undecylglycinnitril-N,N-diacetonitril beträgt 127 g (0,44 mol; 88% d.Th); die Bestimmung erfolgte gaschromatographisch.

### Beispiel 3

### D,L-n-Undecylglycin-N,N-diessigsäure

Das teilkristalline Gemisch aus Beispiel 1 wird im Stahlautoklaven in 400 ml Ethanol vorgelegt. Dazu werden 536 g (2,68 mol) einer 20%igen Natronlauge getropft, und es wird 1 h bei 30°C gerührt. Der Druckrührbehälter wird verschlossen, bevor auf 100°C erhitzt und 6 h bei dieser Temperatur und einem Druck von ca. 4 bar weiterverseift wird. Nach Abkühlen auf Raumtemperatur und Entspannen auf Normaldruck werden die flüchtigen Anteile abdestilliert und der Rückstand in Wasser aufgenommen. Mit konzentrierter Salzsäure wird auf pH = 2 gestellt und der sich bildende Niederschlag durch Filtration isoliert. Es werden 231 g (0,64 mol) 95 %iger D,L-n-Undecylglycindiessigsäure mit einem Calciumbindevermögen von 2,75 mmol/g erhalten, entsprechend einer Ausbeute von 96% d.Th. (85% d.Th. bezogen auf eingesetztes Iminodiacetonitril).

Anstelle des Gemisches aus Beispiel 1 kann auch das Gemisch aus Beispiel 2 eingesetzt werden.

## Patentansprüche

1. Verfahren zur Herstellung von Verbindungen der allgemeinen Formel IIb 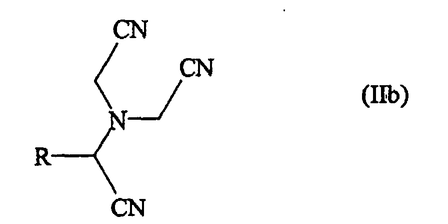 in der R fiir C₆ bis C₃₀-Alkyl oder C₆ bis C₃₀-Alkenyl, welche zusätzlich als Substituenten bis zu 5 Hydroxylgruppen, Formylgruppen, C₁ bis C₄-Alkoxygruppen, Phenoxygruppen oder C₁ bis C₄-Alkoxycarbonylgruppen tragen können und durch bis zu 5 nicht benachbarte Sauerstoffatome unterbrochen sein können, oder Alkoxylat-Gruppierungen der Formel -(CH₂)ₖ-O-(A¹O)ₘ-(A²O)ₙ - Y steht, in der A¹ und A² unabhängig voneinander 1,2-Alkylengruppen mit 2 bis 4 C-Atomen bezeichnen, Y Wasserstoff, C₁ bis C₁₂-Alkyl, Phenyl oder C₁- bis C₄-Alkoxycarbonyl bedeuten und k fiir die Zahl 1, 2 oder 3 sowie m und n jeweils für Zahlen von 0 bis 50 stehen, wobei die Summe aus m + n mindestens 4 betragen muß,
durch Umsetzung von Iminodiacetonitril mit Aldehyden der allgemeinen Formel R-CHO und HCN oder Alkalicyaniden, **dadurch gekennzeichnet, daß** das Verfahren
a) ohne Mitverwendung eines organischen Lösungsmittels durch Umsetzung in Substanz und in Anwesenheit einer Lewis- oder Brönsted-Säure durchgeführt wird.

2. Verfahren zur Herstellung von Verbindungen der allgemeinen Formel IX 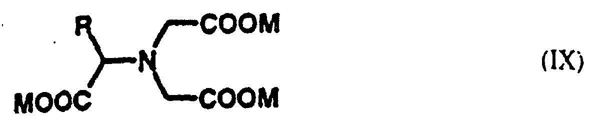 in der R die in Anspruch 1 angegebene Bedeutung hat und M Wasserstoff, Alkalimetall, Erdalkalimetall, Ammonium oder substituiertes Ammonium in den entsprechend stöchiometrischen Mengen bedeutet,
durch Umsetzung von Verbindungen der allgemeinen Formel IIb, wie sie in Anspruch 1 definiert sind und die nach einem Verfahren gemäß Anspruch 1 hergestellt wurden, mit Alkalihydroxidlösungen bei einem Druck im Bereich von 1 bis 40 bar und gegebenenfalls mit Lösungen, die Ionen von M enthalten.

## Claims

1. A process for preparing compounds of the formula IIb 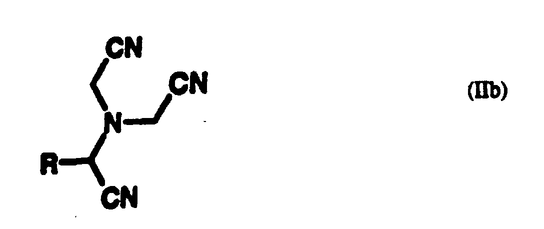 where R is C₆-C₃₀-alkyl or C₆-C₃₀-alkenyl, which may additionally have upto 5 hydroxyl groups, formyl groups, C₁-C₄-alkoxy groups, phenoxy groups or C₁-C₄-alkoxycarbonyl groups as substituents and may be interrupted by upto 5 nonadjacent oxygen atoms, or alkoxylate groups of the formula -(CH₂)ₖ-O-(A¹O)ₘ-(A²O)ₙ-Y where A¹ and A² are, independently of one another, 1,2-alkylene groups having 2 to 4 carbon atoms, Y is hydrogen, C₁-C₁₂-alkyl, phenyl or C₁-C₄-alkoxycarbonyl, and k is 1, 2 or 3, and m and n are each numbers from 0 to 50, and the total of m + n must be at least 4,
by reacting iminodiacetonitrile with aldehydes of the formula R-CHO and HCN or alkali metal cyanides, the process being carried out
a) in the absence of an organic solvent and of any other diluent, and in the presence of a Lewis or Brönsted acid.

2. A process for preparing compounds of the formula IX 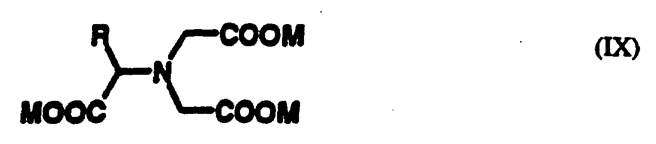 where R has the meaning stated in claim 1, and M is hydrogen, alkali metal, alkaline earth metal, ammonium or substituted ammonium in the appropriately stoichiometric amounts,
by reacting compounds of the formula IIb as are defined in claim 1 and have been prepared by a process as claimed in claim 1, with alkali metal hydroxide solutions under a pressure in the range from 1 to 40 bar and, where appropriate, with solutions which contain ions of M.

## Revendications

1. Procédé de préparation de composés de formule générale IIb 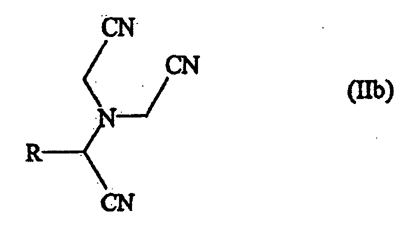 dans laquelle R représente un groupe alkyle en C₆ à C₃₀ ou alcényle en C₆ à C₃₀, lesquels peuvent porter, en plus, en tant que substituants, jusqu'à 5 groupes hydroxyle, groupes formyle, groupes alcoxy en C₁ à C₄, groupes phénoxy ou groupes (alcoxy en C₁ à C₄)carbonyle et peuvent être interrompus par jusqu'à 5 atomes d'azote non vicinaux, ou représente des groupements alcoxylate de formule -(CH₂)ₖ-O-(A¹O)ₘ-(A²O)ₙ-Y, dans laquelle A¹ et A², indépendamment l'un de l'autre, représentent des groupes 1,2-alkylène possédant de 2 à 4 atomes de carbone, Y représente un atome d'hydrogène, un groupe alkyle en C₁ à C₁₂, phényle ou (alcoxy en C₁ à C₄)carbonyle et k représente un nombre valant 1, 2 ou 3, et m et n représentent chacun des nombres de 0 à 50, la somme de m + n devant être égale au moins à 4, par réaction de l'iminodiacétonitrile avec des aldéhydes de formule générale R-CHO et HCN ou des cyanures de métal alcalin, **caractérisé en ce que** l'on réalise le procédé :
a) sans utilisation conjointe d'un solvant organique, au moyen d'une réaction dans une substance et en présence d'un acide de Lewis ou de Brönsted.

2. Procédé de préparation de composés de formule générale IX 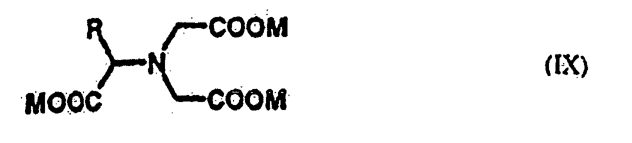 dans laquelle R a la signification indiquée dans la revendication 1 et M représente un atome d'hydrogène, de métal alcalin, de métal alcalino-terreux, d'ammonium ou d'ammonium substitué dans les quantités stoechiométriques correspondantes,
par réaction de composés de formule générale IIb, tels qu'ils sont définis dans la revendication 1 et qui ont été produits selon un procédé selon la revendication 1, avec des solutions d'hydroxydes de métal alcalin, sous une pression dans la gamme de 1 à 40 bars, et éventuellement avec des solutions contenant des ions de M.
